(19) Europäisches Patentamt
European Patent Office
Office européen des brevets

(11) Publication number: 0 250 143 A1

# (12) EUROPEAN PATENT APPLICATION

(21) Application number: 87305067.8

(51) Int. Cl.[4]: C07K 5/06 , A61K 37/02

(22) Date of filing: 09.06.87

(30) Priority: 12.06.86 JP 137741/86

(43) Date of publication of application: 23.12.87 Bulletin 87/52

(84) Designated Contracting States: AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Yoshioka, Kouichi
5-7, Oharano-Kamisatotorimicho Nishikyo-ku
Kyoto 610-11(JP)
Inventor: Harada, Setsuo
3-31, Seiwadainishi 2-chome
Kawanishi Hyogo 666-01(JP)
Inventor: Ochiai, Michihiko
23-13, Senriyamahigashi 1-chome
Suita Osaka 565(JP)

(74) Representative: Lewin, John Harvey et al
Elkington and Fife High Holborn House 52/54
High Holborn
London WC1V 6SH(GB)

## (54) Isoxazolidinone derivatives and production thereof.

(57) The present invention provides a compound of the formula;

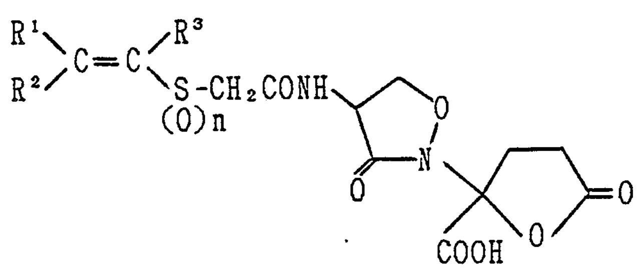

(I)

wherein $R^1$, $R^2$ and $R^3$ are independently hydrogen, halogen, cyano or carbamoyl, and n is an integer of 0, 1 or 2, a salt thereof and ester thereof.

Compound (I) of this invention, its salt and ester have excellent antimicrobial activities, which can be used as antimicrobial agents or therapeutic agents of bacterial infections.

EP 0 250 143 A1

## Isoxazolidinone Derivatives and Production thereof

This invention relates to novel 2-(4-substituted vinylthioacetamido-3-oxo-2-isoxazolidinyl)-5-oxo-2-tetrahydrofurancarboxylic acid, salts thereof and esters thereof, and to methods of preparing them.

Recently, a novel antibiotic TAN-588 (hereinafter referred to in some instances briefly as "TAN-588") exhibiting antimicrobial activity against gram-positive and gram-negative bacterial was harvested from a new species of microorganisms belonging to the genera Empedobacter and Lysobacter as isolated from soil. Thereafter, it was found that the antibiotic TAN-588 had a peculiar skeleton shown as 2-[(4S)-4-acetamido-3-oxo-2-isoxazolidinyl]-5-oxo-2-tetrahydrofurancarboxylic acid.

The present invention aims at preparing novel 2-(4-substituted vinylthioacetamido-3-oxo-2-isoxazolidinyl)-5-oxo-2-tetrahydrofurancarboxylic acid derivatives exhibiting useful antimicrobial activities

The present inventors synthesized derivatives of the antibiotic TAN-588 exhibiting antimicrobial activity, and as a result, it was found that the said derivatives possess excellent antimicrobial activity. The finding was followed by further research, leading to the completion of this invention.

This invention is to provide compounds representable by the formula;

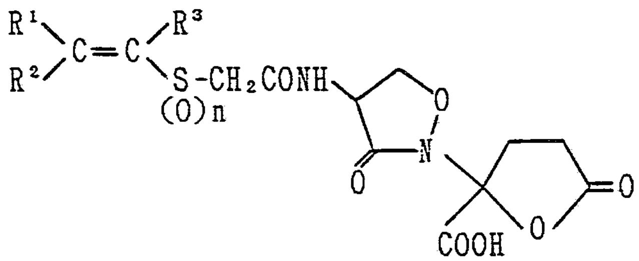

(I)

[wherein $R^1$ , $R^2$ and $R^3$ independently stand for hydrogen, halogen, cyano or carbamoyl, and n denotes an integer of 0, 1 or 2], salts thereof and esters thereof, and to provide methods of preparing the compound (I), salts thereof and esters thereof, characterized by allowing a compound representable by the formula;

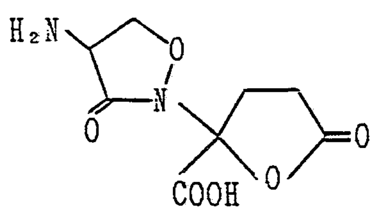

(II)

a salt thereof or an ester thereof to react with a compound representable by the formula;

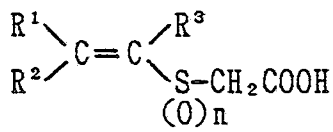

(III)

[wherein $R^1$, $R^2$, $R^3$ and n are of the same meaning as defined above] or its reactive derivative at the carboxyl group, followed by, when desired, subjecting the reaction product to oxidation.

More detailed description of the compound (I) will be given as follows.

The halogen shown by $R^1$, $R^2$ and $R^3$ is exemplified by fluorine, chlorine, bromine or iodine.

The carbamoyl shown by $R^1$, $R^2$ and $R^3$ may have one or two substituents as exemplified by a lower ($C_{1-6}$)alkyl, optionally substituted phenyl or aralkyl(benzyl, phenethyl, etc.) or may form an N-containing 5-to 7-membered ring (pyrrolidine, piperidine, morpholine, N-methylpiperazine, hexamethyleneimine, etc.) together with the nitrogen atom of carbamoyl.

The sulphur atom may be in the form of sulfide (n=0), sulfoxide (n=1) or sulfone (n=2).

The substituted ethylene may take the form of a geometrical isomer depending on the state of substitution and substituents, and, in that case, the isomer may be E-or Z-isomer.

The compound (I) may form a salt at its carboxyl group, as exemplified by pharmacologically acceptable salts including those with an alkali metal (sodium, potassium, etc.) or an alkaline earth metal (calcium, etc.).

The compound (I) may form an ester at its carboxyl group. The ester residue includes an optionally substituted 1-10C alkyl,alkenyl, aryl, cycloalkyl, heterocyclic ring, silyl, etc.

Practical embodiments of the ester are exemplified by methylester, ethylester, n-propylester, isopropylester, t-butylester, t-amylester, benzylester, 4-bromobenzylester, 4-nitrobenzylester, 2-nitrobenzylester, 3,5-dinitrobenzylester, 4-methoxybenzylester, benzhydrylester, phenacylester, 4-bromophenacylester, phenylester, 4-nitrophenylester, methoxymethylester, methoxyethoxymethylester, ethoxymethylester, benzyloxymethylester, acetoxymethylester, pivaloyloxymethylester, 2-methylsulfonylethylester, 2-trimethylsilylethylester, methylthiomethylester, tritylester, 2,2,2-trichloroethylester, 2-iodoethylester, cyclohexylester, cyclopentylester, allylester, cinnamylester, 4-picolylester, 2-tetrahydropyranylester, 2-tetrahydrofuranylester, trimethylsilylester, t-butyldimethylsilylester, t-butyldiphenylsilylester, acetylmethylester, 4-nitrobenzoylmethylester, 4-mesylbenzoylmethylester, phthalimidomethylester, propionyloxymethylester, 1,1-dimethylpropylester, 3-methyl-3-butenylester, succinimidomethylester, 3,5-di-t-butyl-4-hydroxybenzylester, mesylmethylester, benzenesulfonylmethylester, phenylthiomethylester, iminomethylaminoethylester, 1-iminoethylaminoethylester, dimethylaminoethylester, pyridine-1-oxido-2-methylester, methylsulfinylmethylester, bis-(4-methoxyphenyl)methylester, 2-cyano-1,1-dimethylethylester, t-butyloxycarbonylmethylester, benzoylaminomethylester, 1-acetoxyethylester, 1-isobutyryloxyethylester, 1-ethoxycarbonyloxyethylester, 1-cyclohexyloxycarbonyloxyethylester, phthalideester (1,3-dihydro-3-oxo-1-isobenzofuranylester), 4-t-butylbenzylester, 5-indanylester, 5-methyl-2-oxo-1,3-dioxolen-4-ylmethylester, 5-t-butyl-2-oxo-1,3-dioxolen-4-ylmethylester, etc.

Among the compounds (I), those having substitutents at one or two of $R^1$, $R^2$ and $R^3$ are preferable. Preferable substituents are a combination of one or two halogens (especially chlorine), one halogen (especially fluorine) and one carbamoyl (especailly unsubstituted carbamoyl), one cyano, etc.

Also, those wherein either one of $R^1$ and $R^2$ is carbamoyl and the other is fluorine, and, when $R^3$ is H, $R^1$ is carbamoyl and $R^2$ is fluorine (Z-isomer) are preferable.

As the sulphur atom, sulfide ($n = 0$) is especially preferable.

The carboxyl group of compound (I) is preferably in the form of a salt or ester. In the case of a salt, the salt with an alkali metal (especially sodium) is preferable, and, in the case of an ester, the ester whose residual group is carbonyloxy-substituted-methylene (when further substituted, methylene becomes methine) [e.g. pivaloyloxymethylester, 1-(ethoxycarbonyloxy)ethylester, 1-(cyclohexyloxycarbonyloxy)-ethylester, 1,3-dihydro-3-oxo-1-isobenzofuranylester, etc.] is preferable.

The method of preparing the compound (I) is schematically shown as follows, for example :

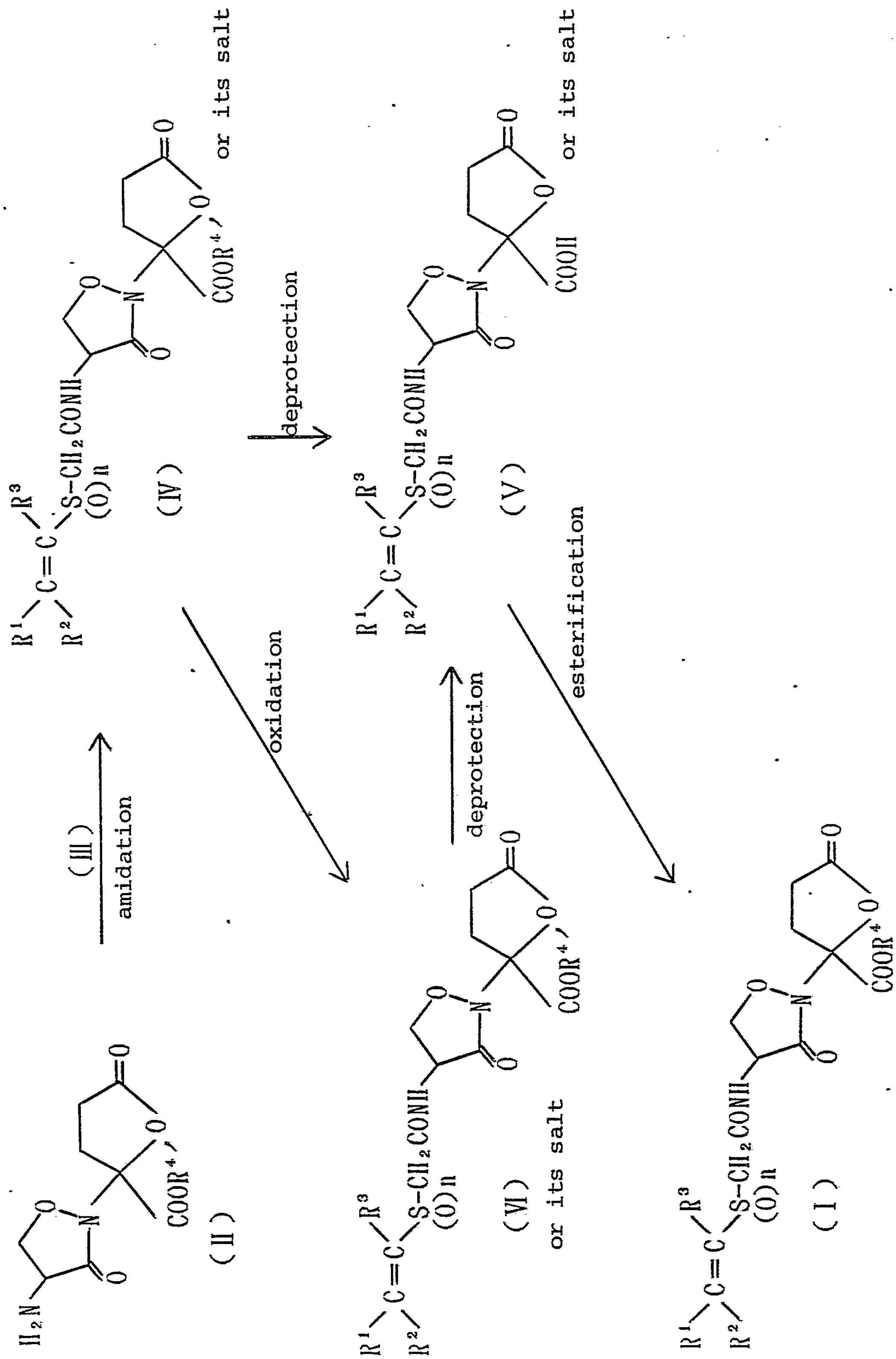


[wherein $R^1$, $R^2$, $R^3$ and n are of the same meaning as defined above, $R^{4'}$ stands for a one forming an intramolecular salt or a carboxyl-protective group, and $R^4$ stands for an ester residue]

The carboxyl-protective group of $R^{4'}$ may be per se known ones as exemplified by benzhydryl, p-nitrobenzyl, etc.

When oxidation is required, n in the starting material is n-1 or n-2.

The method of preparing the compound (I) of this invention is described in detail as follows.

Amidation for preparing the compound (IV) by allowing a compound (II) to react with a compound (III) can be conducted usually in a solvent. A compound (III) can be subjected to the reaction as a free acid or a reactive derivative at the carboxyl group. In the case of a free acid, the reaction with a compound (II) can be conducted in the presence of a condensing agent. The condensing agent is exemplified by carbodiimides such as N,N'-dicyclohexylcarbodiimide, carbonyldiimidazole, 1-ethoxycarbonyl-2-ethoxy-1,2-dihydroxyquinoline, diphenylphophorylazide, etc. As the reactive derivatives at carboxyl group, use is made of, for example, acid halides, acid anhydrides, amido compounds, active ester, active thioesters, etc. These reactive derivatives are specifically described as follows.

1) Acid halides :

As the acid halide employable herein, use is made of, for example, acid chlorides, acid bromides, etc.

2) Acid anhydrides :

As the acid anhydride employable herein, use is made of, for example, mono-alkyl carbonic acid mixed acid anhydrides, mixed acid anhydrides comprising aliphatic carboxylic acids (e.g. acetic acid, pivalic acid, valeric acid, isovaleric acid, trichloroacetic acid, etc.), mixed acid anhydrides comprising aromatic carboxylic acids (e.g. benzoic acid, etc.) or symmetric type acid anhydrides.

3) Amide compounds :

As the amide compound employable herein, use is made of, for example, compounds having an acyl group attached to the nitrogen in the ring, such as pyrazole, imidazole, 4-substituted imidazoles, dimethylpyrazole, benzotriazole, etc.

4) Active esters :

As the active ester, use is made of, for example, esters such as methyl ester, ethyl ester, methoxymethyl ester, propargyl ester, 4-nitrophenyl ester, 2,4-dinitrophenyl ester, trichlorophenyl ester, pentachlorophenyl ester, mesylphenyl ester, etc., as well as esters formed with 1-hydroxy-1H-2-pyridone, N-hydroxysuccinimide, 1-hydroxybenzotriazole, N-hydroxy-5-norbornene-2,3-dicarboxylic acid imide, N-hydroxyphthalimide, etc.

5) Active thioesters :

As the active thioester, use is made of, for example, thioesters formed with heterocyclic thiols such as 2-pyridylthiol, 2-benzothiazolylthiol, etc.

Various kinds of reactive derivatives as described above are suitably selected depending on the type of carboxylic acids.

This reaction is in some instances carried out in the presence of a base. As the base, use is made of, for example, aliphatic tertiary amines (e.g. trimethylamine, triethylamine, tripropylamine, tri-n-butylamine, etc.), tertiary amines such as N-methylpiperidine, N-methylpyrrolidine, cyclohexyldimethylamine, N-methylmorpholine, etc., dialkylamines di-n-butylamine, diisobutylamine, dicyclohexylamine, etc., aromatic amines such as pyridine, lutidine, $\gamma$-collidine, etc., hydroxides or carbonates of an alkali metal such as lithium, sodium, potassium, etc. and those of an alkaline earth metal such as calcium, magnesium, etc., etc.

In this procedure, the reactive derivative of carboxylic acid is normally used at a ratio of about 1 mole per mole of the compound (II), but can be employed in excess, unless it affects the reaction adversely. In the case of using a base, the amount is normally about 1 to 30 moles per mole of the starting compound (II), preferably about 1 to 10 moles, varying with the types of the starting compound (II) used, the kinds of reactive derivative of carboxylic acid then employed and other reaction conditions. This reaction is carried out normally in a solvent. As the solvent, use is made of, for example, conventional solvents, either alone or as a mixture, such as ethers e.g. dioxane, tetrahydrofuran, diethyl ether, diisopropyl ether, propylene oxide,

butylene oxide, etc., esters e.g. ethyl acetate, ethyl formate, etc., halogenated hydrocarbons e.g. chloroform, dichloromethane, 1,2-dichloroethane, 1,1,1-trichloroethane, etc., hydrocarbons e.g. benzene, toluene, n-hexane, etc., amides e.g. N,N-dimethylformamide, N,N-dimethylacetamide, etc. or nitriles e.g. acetonitrile, etc., etc. Among the above-mentioned bases, the liquid ones can also be used to allow them to serve dual purposes of the base and solvent. The reaction temperature is not specifically limited, as far as the reaction proceeds, but the reaction is conducted normally at about -50°C to 150°C, preferably about -30°C to 80°C. The reaction goes to termination normally within several ten minutes to several ten hours, varying with the starting material and base then used, the reaction temperature and the kind of solvent, but in some instances it requires several ten days.

In the compound (IV), $R^{4'}$ is subjected to deprotection reaction, upon necessity, to give the compound (V). As the method of removing the said protective group, a suitable procedure is selected from conventional ones such as the procedure with acid or procedure by means of reduction under such conditions as not exerting influences upon the reaction system. In the case of the procedure with acid, as the acid, use is made of, for example, inorganic acids such as hydrochloric acid, sulfuric acid, phosphoric acid, etc., organic acids such as formic acid, acetic acid, trifluoroacetic acid, propionic acid, etc., as well as acidic ion exchange resins, varying with reaction conditions. Especially, use of trifluoroacetic acid in the presence of anisole is preferable.

In the case of a procedure resorting to reduction, any conditions under which the vinyl group is not reduced can be employed, and, for example, the procedure of using a metal such as tin, zinc, etc. or a metal compound such as chromium dichloride, chromium acetate, etc. and an acid such as organic or inorganic acids e.g. acetic acid, propionic acid, hydrochloric acid, etc., the procedure of reducing in the presence of a metal catalyst for catalytic reduction, etc. can be employed. The catalyst to be used in the procedure of catalytic reduction includes, for example, platinum catalysts such as platinum lead, platinum sponge, platinum black, platinum oxide, colloidal platinum, etc., palladium catalysts such as palladium sponge, palladium black, palladium oxide, palladium barium sulfate, palladium barium carbonate, palladium carbon, palladium silica gel, colloidal palladium, etc., reduced nickel, nickel oxide, Raney nickel, Urushibara nickel, etc. In the case of the procedure through reduction with a metal and an acid, use is made of a metal such as iron, chromium etc. and an inorganic acid such as hydrochloric acid, etc. or an organic acid such as formic acid, acetic acid, propionic acid, etc. The procedure by means of reduction is normally carried out in a solvent, and in the case of the procedure by means of catalytic reduction, for example, are frequently used alcohols such as methanol, ethanol, propyl alcohol, isopropyl alcohol, etc., ethyl acetate, and the like. In the case of the procedure with a metal and an acid, use is frequently made of water, acetone, etc., and, the acid used is in liquid state, the acid itself can be used as a solvent.

The reaction temperature in the procedure with an acid and in the procedure by means of reduction is normally within the range of under cooling to room temperature.

The compound (IV) (wherein n = 0, 1) is, upon necessity, subejcted to oxidation to give the compound (VI) (wherein n = 1 or 2). As the oxidizing agent, use is preferably made of mild ones, exemplified by perbenzoic acid, ozone, hydrogen peroxide, selenium dioxide, sodium m-periodate, etc., and use of a substituted perbenzoic acid such as m-chloroperbenzoic acid, etc. is preferable. The reaction is conducted in a solvent at -50° to 50°C for several minutes to several hours. As the solvent, use is made of water, alcohol, dichloromethane, etc. The amount of the oxidizing agent ranges from equivalent relative to the compound (IV) to an excess amount unless it affects the reaction system adversely.

The compound (VI) is subjected to the above-mentioned deprotection reaction to give the compound (V).

The compound (V) is subjected, upon necessity, to esterification to give the compound (I) (wherein $R^4$ does not stands for hydrogen or a salt).

The esterification can be conducted by, for example, the following procedures.

1) The compound (V) is allowed to react with a diazoalkane, such as diazomethane, phenyldiazomethane, diphenyldiazomethane, etc., in a solvent such as tetrahydrofuran, dioxane, ethyl acetate, acetonitrile, etc., at about 0°C to its refluxing temperature for about 2 minutes to 2 hours.

2) An alkali metal salt of the compound (V) is allowed to react with an activated alkyl halide such as methyl iodide, benzyl bromide, p-nitrobenzyl bromide, m-phenoxybenzyl bromide, p-t-butylbenzyl bromide, pivaloyloxymethyl chloride, etc. With reference to the suitable reaction conditions, the reaction is allowed to proceed in a solvent such as dimethylformamide, dimethylacetamide, hexamethylphosphoramide, etc., at about 0°C to 60°C for about 2 minutes to 4 hours. Coexistence of triethylamine, etc. in this reaction solution does not affect adversely the reaction.

3) The compound (V) is allowed to react with an alcohol such as methanol, ethanol, benzyl alcohol, etc. This reaction is conducted in the presence of a carbodiimide condensing agent such as dicyclohexylcarbodiimide at about 0°C to the refluxing temperature of the solvent used for about 15 minutes to 18 hours. As the solvent, use is made of chloroform, dichloromethane, dichloroethane, etc.

4) An acid anhydride of the compound (V) formed by allowing the compound (V) to react with an acid chloride such as ethyl chloroformate, benzyl chloroformate, etc. is allowed to react with an alcohol such as those mentioned in 3) above under the reaction conditions described in 3) above. This anhydride is obtainable by allowing the compound (V) to react with an acid chloride in a solvent such as tetrahydrofuran, dichloromethane, etc., at 25°C to the refluxing temperature for about 15 minutes to 10 hours.

5) The compound (V) is allowed to react with a silylating agent such as trimethylsilyl chloride or t-butyldimethylsilyl chloride, in the copresence of, for example, triethylamine in a solvent such as dichloromethane, chloroform, tetrahydrofuran, etc. at about 0°C to the refluxing temperature for about 15 minutes to 16 hours.

In the method of this invention, the compounds (II) can be prepared by using as starting materials the antibiotic TAN-588 (2-[(4S)-4-acetamido-3-oxo-2-isoxazolidinyl]-5-oxo-2-tetrahydrofurancarboxylic acid), its salts, its esters or their N-deacetyl derivatives.

These starting compounds are all known ones, and the methods of preparing them as well as their physico-chemical properties are described in EP Publication (laid open) No. 157544.

The starting compounds representable by (III) are also known ones, and the methods of preparing them and so on are described in EP Publication (laid open) No.107928, etc.

The objective compound (I) thus obtained can be isolated and purified by the per se known means such as concentration, phasic transfer, solvent extraction, lyophilization, crystallization, recrystallization, fractional distillation, chromatography, etc.

The presence of two asymmetric carbons in the basic skeleton allows theoretically the objective compound (I) to exist in four kinds of stereoisomers, and their individual stereoisomers and mixtures thereof fall into the scope of this invention. Similarly, occurrence of any asymmetric carbon in the ester residue or isomers in the sulfoxide group, and mixture thereof are also included in the scope of this invention. In cases where the above-described reaction produces these isomers as a mixture, their individual stereoisomers can be isolated by conventional methods such as various chromatographic procedures, recrystallization, etc.

The compound (I) of this invention can, in some instances, act on bases to form salts.

The compound (I) of this invention, when obtained in the free form, may be allowed to form salts by means of a conventional procedure, or the compound (I) obtained in the form of a salt may be converted into the free form by using a conventional means.

And, the compound (I), in some instances, forms an intramolecular salt, and this salt is included in the scope of this invention.

The stereoisomers of the compound (I), either alone or as a mixture, can be used as medicinal preparations.

The compound (I) thus obtained is useful as drugs, for example, having antimicrobial activity against certain species of gram-positive and gram-negative bacteria.

Biological properties of the compound (I) are decribed in the following. Typical compounds of the compound (I) demonstrate antimicrobial spectra against various microorganisms as shown in Table 1.

Table 1 : [Minimum growth inhibitory concentration*(μg/mℓ)]

| Compound** / Test microorganisms | 1b | 6b | 9b | 10b | 15 | Control |
|---|---|---|---|---|---|---|
| Staphylococcus aureus FDA 209P | 0.2 | <0.1 | <0.1 | 0.78 | 0.78 | 100 |
| Escherichia coli NIHJ JC-2 | 3.13 | 1.56 | 6.25 | 6.25 | 6.25 | >100 |
| Klebsiella pneumoniae DT | 3.13 | 3.13 | 3.13 | 6.25 | 6.25 | >100 |
| Pseudomonas aeruginosa IFO 3455 | >100 | >100 | >100 | >100 | >100 | >100 |

* Medium : Trypticase soy agar
Amount of a bacterium inoculated : $10^8$ CFU/mℓ

** The compound number corresponds to that referred to in working examples. As the control, a deacetyl compound of the known antibiotic TAN-588 (cf. Example 11 in EP Publication (laid open) No. 157544 was employed.

As described above, the compound (I) of this invention, its salts and esters thereof show antimicrobial activity against some species of gram-positive and gram-negative bacteria, and, in addition, are of low toxicity. They can, therefore, be used as an antimicrobial agent or as a therapeutic agent for bacterial infections, for the treatment of bacterial infections (e.g. respiratory tract infections, urinary tract infections, pyogenic diseases, bile duct infections, intratestinal infections, obsteric and gynecological infections, surgical infections, etc.) in mammal (e.g., mouse, rat, dog, cattle, pig, human being) caused by infections with bacteria.

The daily dosage of the compound (I) or its salts is in an amout of about 2 to 100 mg/kg as the compound (I), more preferably about 5 to 40 mg/kg.

For administration of the compound (I), the compound (I) or its pharmacologically acceptable salt can be formulated by conventional means with suitable pharmacologically acceptable carrier, excipient or diluent into such dosage forms as tablet, granule, capsule or elixir to administer orally, and also can be processed into injectable solution by conventional means, followed by incorporation into a sterile carrier prepared by conventional means to administer parenterally.

In producing the above-described oral pharmaceutical preparations, such as tablets, there can suitably be formulated binding agents (e.g. hydroxypropylcellulose, hydroxypropylmethyl cellulose, macrogol, etc.), disintegrating agents (e.g. starch, carboxymethylcellulose calcium, etc.), excipients (e.g. lactose, starch, etc.), lubricants (e.g. magnesium stearate, talc, etc.) and the like.

In manufacturing non-oral or parenteral pharmaceutical preparations, such as injectable solutions, there can suitably be formulated isotonizing agents (e.g. glucose, D-sorbitol, D-mannitol, sodium chloride, etc.), preservatives (e.g. benzyl alcohol, chlorobutanol, methyl p-oxybenzoate, propyl p-oxybenzoate, etc.), buffering agents (e.g. phosphate buffers, sodium acetate buffer, etc.) and the like.

## Examples

The following examples are given to illustrate the present invention in further detail, but they are not intended to limit the invention thereto.

Abbreviations used in NMR denote as follows :

s : singlet d : doublet dd : doublet of doublets

t : triplet q : quartet m : multiplet b : broad

Example 1

Production of sodium 2-{(4S)-4-[(Z)-2-chlorovinylthioacetamido]-3-oxo-2-isoxazolidinyl}-5-oxo-2-tetrahydrofurancarboxylate [Compound (1b)] :

(a) To a solution of 152 mg of (Z)-2-chlorovinylthioacetic acid in 6 mℓ of N,N-dimethylformamide (DMF) were added 230 mg of N,N'-dicyclohexylcarbodiimide (DCC) and 150 mg of 1-hydroxybenzotriazole (HOBT). The mixture was stirred at room temperature for 10 minutes, to which was added 300 mg of benzhydrylester of a deacetyl compound of TAN-588 (benzhydryl 2-[(4S)-4-amino-3-oxo-2-isoxazolidinyl]-5-oxo-2-tetrahydrofurancarboxylate), followed by stirring for one hour. The reaction solution was diluted with ethyl acetate, and precipitates then formed were filtered off. The filtrate was washed with water and dried ($Na_2SO_4$), then the solvent was distilled off. The residue was subjected to a silica gel column chromatography, followed by elution with ethyl acetate - n-hexane (4:3) to give 342 mg of benzhydryl 2-{(4S)-4-[(Z)-2-chlorovinylthioacetamido]-3-oxo-2-isoxazolidinyl}5-oxo-2-tetrahydrofurancarboxylate [Compound (1a)].

IR$\nu_{max}^{KBr}$ $cm^{-1}$:3340, 1780, 1720, 1680, 1520, 1180, 1050
NMR(90MHz,$CDCl_3$)δ: 2.32-3.40(4H,m), 3.37(2H,s), 3.91-4.20(1H,m), 4.52-5.01(2H,m), 6.12(1H,d,J=6Hz), 6.33(1H,d,J=6Hz), 6.82(1H,s), 7.22-7.43(10H,m)

(b) A solution of 200 mg of Compound (1a) as obtained above in 15 mℓ of dichloromethane was cooled at -10° to -15°C, to which were added 0.35 mℓ of trifluoroacetic acid and 0.29 mℓ of anisole, followed by stirring for 5 hours. The mixture was concentrated to dryness under reduced pressure, which was washed with n-hexane, followed by dissolving in ethyl acetate. To the solution was added 3 mℓ of water containing 40 mg of sodium hydrogencarbonate. The aqueous layer was separated and subjected to a column chromatography using XAD-2 (produced by Rohm & Haas Co., U.S.A.). Fractions eluted with 10% ethanol were combined and concentrated, followed by lypophilizing to give 85 mg of the subject Compound (1b).

IR$\nu_{max}^{KBr}$ $cm^{-1}$: 3450, 1780, 1720, 1650, 1380,1190, 1110, 1030
NMR(100MHz,$D_2O$)δ : 2.45-3.30(4H,m), 3.64(2H,s), 4.02-4.33(1H,m), 4.61-4.82(1H,m), 4.97-5.24(1H,m), 6.33-(1H,d,J=6Hz), 6.58(1H,d,J=6Hz)

Example 2

Production of pivaloyloxymethyl 2-{(4S)-[(Z)-2-chlorovinylthioacetamido]-3-oxo-2-isoxazolidinyl)-5-oxo-2-tetrahydrofurancarboxylate [Compound (2)]:

To a solution of 237 mg of Comound (1b) obtained in Example 1 in 5 mℓ of DMF was added 0.18 mℓ of pivaloyoxymethylchloride, and the mixture was stirred at room temperature for 24 hours. The reaction solution was poured into water, followed by extraction with ethyl acetate. The organic layer was washed with water, which was then dried ($Na_2SO_4$), followed by distilling off the solvent. The residue was subjected to a silica gel column chromatography, eluting with ethyl acetate - n-hexane (2:1) to give 168 mg of the subject Compound (2).

IR$\nu_{max}^{KBr}$ $cm^{-1}$: 3300, 1800, 1750, 1660, 1530, 1180, 1120, 1030
NMR(90MHz,$CDCl_3$)δ : 1.23(9H,s), 2.31-3.39(4H,m), 3.48(2H,s), 4.01-4.55(1H,m), 4.65-5.07(2H,m), 5.83-(2H,s), 6.19(1H,d,J=6Hz), 6.41(1H,d,J=6Hz)

Example 3

Production of 1-[(ethoxycarbonyl)oxy]ethyl-2-{(4S)-4-[(Z)-2-chlorovinylthioacetamido]-3-oxo-2-isoxazolidinyl}-5-oxo-2-tetrahydrofurancarboxylate [Compound (3):

To a solution of 115 mg of Compound (1b) obtained in Example 1 in 2 mℓ of DMF was added 0.2 mℓ of 1-[(ethoxycarbonyl)oxy]ethyl iodide. The mixture was stirred at room temperature for 2.5 hours, followed by workup in a manner similar to Example 2 to give 88 mg of the subject Compound (3).

IR $\nu_{max}^{KBr}$ $cm^{-1}$: 3350, 1800, 1760, 1680, 1530, 1180, 1160, 1050
NMR(90MHz,$CDCl_3$)δ : 1.29(3H,t,J=7Hz), 2.53(3H,d,J=7Hz), 2.38-3.48(4H,m), 3.42(2H,s), 3.98-4.31(3H,m), 4.62-5.12(2H,m), 6.16(1H,d,J=6Hz), 6.38(1H,d,J=6Hz), 6.78(1H,q,J=7,12Hz)

Example 4

Production of 1-[(cyclohexyloxycarbonyl)oxy]ethyl 2-{(4S)-4 -[(Z)-2-chlorovinylthioacetamido]-3-oxo-2-isoxazolidinyl}-5-oxo-2-tetrahydrofurancarboxylate [Compound (4)] :

To a solution of 174 mg of Compound (1b) obtained in Example 1 in 4 mℓ of DMF was added 0.3 mℓ of 1-[(cyclohexyloxycarbonyl)oxy]ethyl iodide. The mixture was stirred at room temperature for 24 hours, followed by workup in a manner similar to Example 2 to give 118 mg of the subject Compound (4).

IR $\nu_{max}^{KBr}$ $cm^{-1}$: 3350, 1800, 1760, 1680, 1530, 1180, 1160, 1050
NMR(90MHz,$CDCl_3$)δ : 1.12-1.99(10H,m), 1.55(3H,d,J=6Hz), 2.32-3.53(4H,m), 3.41(2H,s), 3.92-4.20(1H,m), 4.41-4.90(3H,m), 6.11(1H,d,J=6Hz), 6.21(1H,q,J=6,12Hz), 6.72(1H,d,J=6Hz)

Example 5

Production of 1,3-dihydro-3-oxo-1-isobenzofuranyl 2-{(4S)-4-[(Z)-2-chlorovinylthioacetamido]-3-oxo-2-isoxazolidinyl}-5-oxo-2-tetrahydrofurancarboxylate [Compound (5)]:

To a solution of 100 mg of Compound (lb) obtained in Example 1 in 2 mℓ of DMF was added 81 mg of 3-bromophthalide. The mixture was stirred at room temperature for 24 hours, followed by workup in a manner similar to Example 2 to give 81mg of the subject Compound (5).

IR $\nu_{max}^{KBr}$ $cm^{-1}$:3350, 1800-1750, 1670, 1530, 1180, 1050, 980
NMR(90MHz,$CDCl_3$)δ; 2.40-3.41(4H,m), 3.48(2H,s), 4.05-4.31(1H,m), 4.62-5.17(2H,m), 6.19(1H,d,J=6Hz), 6.41(1H,d,J=6Hz), 7.46(1H,s), 7.63-8.01(4H,m)

Example 6

Production of sodium 2-[(4S)-4-(2,2-dichlorovinylthioacetamido)-3-oxo-2-isoxazolidinyl]-5-oxo-2-tetrahydrofurancarboxylate [Compound (6b)]:

A procedure similar to Example 1 (a), using 187 mg of 2,2-dichlorovinylthioacetic acid and 320 mg of benzhydryl ester of a deacetyl compound of TAN-588, gave 330 mg of benzhydryl 2-[(4S)-4-(2,2-dichlorovinylthioacetamido)-3-oxo-2-isoxazolidinyl]-5-oxo-2-tetrahydrofurancarboxylate [Compound (6a)].

IR $\nu_{max}^{KBr}$ $cm^{-1}$: 3350, 1770, 1720, 1680, 1520, 1180, 1050
NMR (90MHz,$CDCl_3$)δ : 2.32-3.30(4H,m), 3.36,3.40(2H,each s), 3.97-4.22(1H,m), 4.53-5.08(2H,m), 6.40(1H,s), 6.93(1H,s), 7.19-7.55(10H,m)

A procedure similar to Example 1 (b), using 200 mg of Compound (6a), gave 67 mg of the subject Compound (6b).

IR $\nu_{max}^{KBr}$ $cm^{-1}$: 3400, 1780, 1730, 1650, 1380, 1190, 1110, 1030, 910
NMR(90MHz,$D_2O$)δ : 2.36-3.35(4H,m), 3.64(2H,s), 4.07-4.35(1H,m), 4.61-4.82(1H,m), 4.97-5.22(1H,m), 6.70-(1H,s)

Example 7

Production of sodium 2-[(4S)-4-(2,2-dichlorovinylsulfinylacetamido)-3-oxo-2-isoxazolidinyl]-5-oxo-2-tetrahydrofurancarboxylate [Compound (7b)] :

(a) To a solution of 608 mg of Compound (6a) obtained in Example 6(a) in 36 mℓ of dichloromethane was added 265 mg of m-chloro perbenzoic acid (purity 70%). The mixture was stirred at 0°C for 5 minutes and, then, at room temperature for 30 minutes, followed by extraction with ethyl acetate. The organic layer was washed with an aqueous solution of sodium hydrogencarbonate and dried ($Na_2SO_4$). The solvent was distilled off to give 540 mg of benzhydryl 2-[(4S)-4-(2,2-dichlorovinylsulfinylacetamido)-3-oxo-2-isoxazolidinyl] -5-oxo-2-tetrahydrofurancarboxylate [Compound (7a)].

IR$\nu_{max}^{KBr}$ $cm^{-1}$: 3340, 1770, 1730, 1680, 1570, 1180, 1050,
NMR(90MHz,$CDCl_3$)$\delta$ : 2.25-3.60(4H,m), 3.34,3.53(2H,each s), 3.91-4.22(1H,m), 4.49-5.08(2H,m), 7.00(1H,s), 7.04,7.05(1H,each s), 7.29-7.50(10H,m)

(b) A procedure similar to Example 1 (b), using 200 mg of the above Compound (7a), gave 98 mg of the subject Compound (7b).

IR$\nu_{max}^{KBr}$ $cm^{-1}$: 3450, 1780, 1720, 1650, 1380, 1190, 1040, 910
NMR(100MHz,$D_2O$)$\delta$ : 2.42-3.20(4H,m), 4.10-4.39(1H,m), 4.51-4.80(1H,m), 4.11,4.27(2H,each s), 4.92-5.28-(1H,m), 7.24(1H,s)

Example 8

Production of sodium 2-[(4S)-4-(2,2-dichlorovinylsulfonylacetamido)-3-oxo-2-isoxazolidinyl]-5-oxo-2-tetrahydrofurancarboxylate [Compound (8b)] :

(a) To a solution of 340 mg of Compound (7a) obtained in Example 7(a) in 15 mℓ of dichloromethane was added 173 mg of m-chloro perbenzoic acid (purity 70%). The mixture was stirred at 0°C for 5 minutes and, then, at room temperature for 5 hours. The resultant was subjected to workup in a manner similar to Example 7(a) to give 100 mg of benzhydryl 2-[(4S)-4-(2,2-dichlorovinylsulfonylacetamido)-3-oxo-2-isoxazolidinyl]-5-oxo-2-tetrahydrofurancarboxylate [Compound (8a)].

(b) A procedure similar to Example 1 (b), using 100 mg of Compound (8a), gave 45 mg of the subject Compound (8b).

IR$\nu_{max}^{KBr}$ $cm^{-1}$: 3400, 1780, 1720, 1650, 1380, 1330, 1190, 1140, 1030, 920
NMR(90MHz,$D_2O$)$\delta$ : 2.40-3.30(4H,m), 4.10-4.48(1H,m), 4.59-4.88(2H,s, 1H,m), 4.92-5.27(1H,m), 7.28(1H,s)

Example 9

Production of sodium 2-{(4S)-4-[(Z)-1,2-dichlorovinylthioacetamido]-3-oxo-2-isoxazolidinyl}-5-oxo-2-tetrahydrofurancarboxylate [Compound (9b)] :

(a) A procedure similar to Example 1 (a), using 187 mg of (Z)-1,2-dichlorovinylthioacetic acid and 320 mg of benzhydryl ester of a deacetyl compound of TAN-588, gave 358 mg of benzhydryl 2-{(4S)-4-[(Z)-1,2-dichlorovinylthioacetamido]-3-oxo-2-isoxazolidinyl}-5-oxo-2-tetrahydrofurancarboxylate [Compound (9a)].

IR$\nu_{max}^{KBr}$ $cm^{-1}$: 3340, 1770, 1720, 1680, 1520, 1180, 1050
NMR(90MHz,$CDCl_3$)$\delta$ : 2.15-3.52(4H,m), 3.67(2H,s), 3.91-4.20(1H,m), 4.53-4.98(2H,m), 6.50(1H,s), 6.99-(1H,s), 7.21-7.45(10H,m)

(b) A procedure similar to Example 1 (b), using 200 mg of Compound (9a), gave 67 mg of the subject Compound (9b).

IR$\nu_{max}^{KBr}$ $cm^{-1}$: 3400, 1780, 1730, 1650, 1380, 1190, 1110, 1030, 910
NMR(90MHz,$D_2O$)$\delta$ : 2.42-3.32(4H,m), 3.82(2H,s), 4.00-4.34(1H,m), 4.61-4.82(1H,m), 4.85-5.48(1H,m), 6.83-(1H,s)

Example 10

Production of sodium 2-{(4S)-4-[(Z)-1,2-dichlorovinylsulfinylacetamido]-3-oxo-2-isoxazolidynyl}-5-oxo-2-tetrahydrofurancarboxylate [Compound (10b)] :

(a) A procedure similar to Example 7 (a), using 328 mg of Compound (9a) obtained in Example 9 (a), gave 240 mg of benzhydryl 2-{(4S)-4-[(Z)-1,2-dichlorovinylsulfinylacetamido] -3-oxo-2-isoxazolidinyl}-5-oxo-2-tetrahydrofurancarboxylate [Compound (10a)].

IR$\nu_{max}^{KBr}$ $cm^{-1}$: 3340, 1770, 1730, 1680, 1540, 1180, 1050
NMR(90MHz,$CDCl_3$)$\delta$ : 2.18-3.60(4H,m), 3.73,3.79(2H,each s), 3.94-4.20(1H,m), 4.49-5.18(2H,m), 6.75,6.77-(1H,each s), 7.0(1H,s), 7.22-7.53(10H,m)

(b) A procedure similar to Example 1 (b), using 240 mg of Compound (10a), gave 118 mg of the subjet Compound (10b).

IR$\nu_{max}^{KBr}$ $cm^{-1}$: 3450, 1780, 1730, 1650, 1380, 1190, 1110, 1040, 910
NMR(90MHz,$D_2O$)$\delta$ : 2.42-3.24(4H,m), 4.09-4.35(1H,m), 4.18(2H,s), 4.51-4.80(1H,m), 4.92-5.28(1H,m), 7.25-(1H,s)

Example 11

Production of sodium 2-{(4S)-4-[(Z)-2-carbamoyl-2-fluorovinylthioacetamido]-3-oxo-2-isoxazolidinyl}-5-oxo-2-tetrahydrofurancarboxylate [Compound (11b)] :

(a) A procedure similar to Example 1 (a), using 179 mg of (Z)-2-carbamoyl-2-fluorovinylthioacetic acid and 356 mg of benzhydryl ester of a deacetyl compound of TAN-588, gave 358 mg of benzhydryl 2-{(4S)-4-[(Z)-2-carbamoyl-2-fluorovinylthioacetamido]-3-oxo-2-isoxazolidinyl}-5-oxo-2-tetrahydrofurancarboxylate [Compound (11a)].

IR$\nu_{max}^{KBr}$ $cm^{-1}$: 3320, 1770, 1720, 1670, 1620, 1400, 1180, 1050
NMR(90MHz,$CDCl_3$+$d_6$-DMSO)$\delta$ : 2.28-3.42(4H,m), 3.52(2H,s), 3.98-4.25(1H,m), 4.48-5.02(2H,m), 6.99-(1H,s), 6.98-7.50(11H,m)

(b) A procedure similar to Example 1 (b), using 200 mg of the above Compound (11a), gave 90 mg of the subject Compound (11b).

IR$\nu_{max}^{KBr}$ $cm^{-1}$: 3400, 1780, 1720, 1680, 1650, 1400, 1190, 1030
NMR(100MHz,$D_2O$)$\delta$ : 2.43-3.36(4H,m), 3.80(2H,s), 4.13-4.35(1H,m), 4.64-4.83(1H,m), 4.93-5.23(1H,m), 6.94-(1H,d,J=32Hz)

Example 12

Production of pivaloyloxymethyl 2-{(4S)-4-[(Z)-2-carbamoyl-2-fluorovinylthioacetamido]-3-oxo-2-isoxazolidinyl}-5-oxo-2-tetrahydrofurancarboxylate [Compound (12)] :

A procedure similar to Example 2, using 214 mg of Compound (11b) obtained in Example 11 (b), gave 157 mg of the subject Compound (12).

IR$\nu_{max}^{KBr}$ $cm^{-1}$: 3350, 1800, 1740, 1680, 1650, 1400, 1180, 1030
NMR(90MHz,$CDCl_3$)$\delta$ : 1.25(9H,s), 2.34-3.52(4H,m), 3.58(2H,s), 4.05-4.27(1H,m), 4.63-5.17(2H,m), 5.87-(2H,s), 6.26(2H,bs), 7.06(1H,d,J=33Hz), 7.74(1H,m)

Example 13

Production of 1-[(ethoxycarbonyl)oxy]ethyl 2-{(4S)-4-[(Z)-2-carbamoyl-2-fluorovinylthioacetamido]-3-oxo-2-isoxazolidinyl}-5-oxo-2-tetrahydrofurancarboxylate [Compound (13)]:

A procedure similar to Example 11, using 243 mg of Compound (11b) obtained in Example 11 (b), gave 134 mg of the subject Compound (13).

IR$\nu_{max}^{KBr}$ $cm^{-1}$: 3350, 1800, 1760, 1680, 1660, 1400, 1250, 1180, 1030
NMR(90MHz,$CDCl_3$)$\delta$ : 1.29(3H,t,J=7Hz), 2.56(3H,d,J=7Hz), 2.34-3.52(4H,m), 3.59(2H,s), 4.06-4.37(3H,m), 4.71-5.12(2H,m), 6.47(2H,bs), 6.76-6.88(1H,m), 7.06(1H,d,J=33Hz), 7.85(1H,m)

Example 14

Production of sodium 2-{(4S)-4-[(E)-2-cyanovinylthioacetamido]-3-oxo-2-isoxazolidinyl}-5-oxo-2-tetrahydrofurancarboxylate [Compound (14b)] :

(a) A procedure similar to Example 1 (a), using 143 mg of (E)-2-cyanovinylthioacetic acid and 300 mg of benzhydryl ester of a deacetyl compound of TAN-588, gave 284 mg of benzhydryl 2-{(4S)-4-[(E)-2-cyanovinylthioacetamido]-3-oxo-2-isoxazolidinyl}-5-oxo-2-tetrahydrofurancarboxylate [Compound (14a)].

IR$\nu_{max}^{KBr}$ $cm^{-1}$: 3340, 2220, 1770, 1720, 1680, 1570, 1180, 1050
NMR(90MHz,$CDCl_3$)$\delta$ : 2.39-3.32(4H,m), 3.71(2H,s), 3.95-4.27(1H,m), 4.42-5.09(2H,m), 5.39(1H,d,J=16Hz), 6.98(1H,s), 6.38-6.98(10H,m), 6.50(1H,d,J=16Hz)

(b) A procedure similar to Example 1 (a), using 284 mg of Compound (14a) obtained as above, gave I27 mg of the subject Compound (14b).

IR$\nu_{max}^{KBr}$ $cm^{-1}$: 3350, 2220, 1770, 1720, 1650, 1550, 1380, 1190, 1110, 1040
NMR(100MHz,$D_2O$)$\delta$ : 2.46-3.30(4H,m), 3.84(2H,s), 4.12-4.34(1H,m), 4.58-4.86(1H,m), 4.98-5.22(1H,m), 5.54-(1H,d,J=16Hz), 7.70(1H,d,J=16Hz)

Example 15

Production of sodium 2-{(4S)-4-[(Z)-2-cyanovinylthioacetamido]-3-oxo-2-isoxazolidinyl}-5-oxo-2-tetrahydrofurancarboxylate [Compound (15)] :

A procedure similar to Example 14, using 143 mg of (Z)-2-cyanovinylthioacetic acid and 300 mg of benzhydryl ester of a deacetyl compound of TAN-588, gave 105 mg of the subject Compound (15).

IR$\nu_{max}^{KBr}$ $cm^{-1}$: 3350, 2220, 1780, 1720, 1650, 1540, 1380, 1190, 1110, 1040
NMR(100MHz,$D_2O$)$\delta$ : 2.45-3.30(4H,m), 3.84(2H,s), 4.12-4.36(1H,m), 4.56-4.84(1H,m), 4.98-5.22(1H,m), 5.64-(1H,d,J=10Hz), 7.52(1H,d,J=10Hz)

Example 16

Production of 1-[(ethoxycarbonyl)oxy]ethyl 2-{(4S)-4-[(Z)-2-cyanovinylthioacetamido]-3-oxo-2-isoxazolidinyl}-5-oxo-2-tetrahydrofurancarboxylate [Compound (16)] :

A procedure similar to Example 3, using 220 mg of Compound (15) obtained in Example 15, gave 127 mg of the subject Compound (16).

IR$\nu_{max}^{KBr}$ $cm^{-1}$: 3340, 2220, 1800, 1760, 1680, 1540, 1260, 1180, 1050
NMR(90MHz,$CDCl_3$)$\delta$ : 1.29(3H,t,J=7Hz), 1.57(3H,d,J=7Hz), 2.38-3.30(4H,m), 3.63(2H,s), 4.08-4.38(3H,m), 4.59-5.17(2H,m), 5.39(1H,d,J=10Hz), 6.70-6.96(1H,m), 7.35(1H,d,J=10Hz)

Example 17

Using the following ingredients, tablets are produced by the conventional means :

| | |
|---|---|
| Compound (3) as obtained in Example 3 | 300 mg |
| Corn · starch | 50 mg |
| Lactose | 28 mg |
| Hydroxypropylcellulose L | 20 mg |
| Magnesium stearate | 2 mg |
| | 400 mg (per tablet) |

Dosage : 4 to 8 tablets/adult/day after each meal (three time per day)

Example 18

Sterilized vials, 12 mℓ each capacity, are filled with 3 mg equivalent each of Compound (6b) as obtained in Example 6, which are sealed under 50 mmHg. Dissolution with 3 mℓ of distilled water gives an injectable solution.

## Claims

1. A compound of the formula;

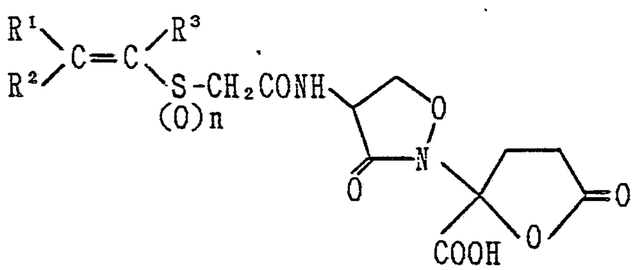

wherein $R^1$, $R^2$ and $R^3$ are independenly hydrogen, halogen, cyano or carbamoyl, and n is an integer of 0, 1 or 2, a salt thereof or an ester thereof.

2. The compound according to claim 1, wherein one or two of $R^1$, $R^2$ and $R^3$ have substituents and the others are hydrogens.

3. The compound according to claim 2, wherein the substituents are a combination of one or two halogens, one halogen and one carbamoyl, or one cyano.

4. The compound according to claim 3, wherein halogen is chlorine or fluorine.

5. The compound according to claim 1, wherein $R^1$ is hydrogen, chlorine, carbamoyl or cyano.

6. The compound according to claim 1, wherein $R^2$ is chlorine, fluorin or cyano.

7. The compound according to claim 1, wherein $R^3$ is hydrogen or chlorine.

8. The compound according to claim 1, wherein $R^1$ is hydrogen or chlorine, $R^2$ is chlorine or cyano and $R^3$ is hydrogen or chlorine.

9. The compound according to claim 1, wherein n is 0.

10. The compound according to claim 1, which is a salt.

11. The compound according to claim 10, wherein the salt is a sodium salt.

12. The compound according to claim 1, which is an ester.

13. The compound according to claim 12, wherein the ester is a carbonyloxy-substituted-methylene ester.

14. The compound according to claim 1, which is sodium 2-{(4S)-4-[(Z)-2-chlorovinylthioacetamido]-3-oxo-2-isoxazolidinyl}-5-oxo-2-tetrahydrofurancarboxylate.

15. The compound according to claim 1, which is sodium 2-[(4S)-4-(2,2-dichlonovinylthioacetamido)-3-oxo-2-isoxazolidinyl]-5-oxo-2-tetrahydrofurancarboxylate.

16. The compound according to claim 1, which is sodium 2-{(4S)-4-[(Z)-1,2-dichlorovinylthioacetamido]-3-oxo-2-isoxazolidinyl}-5-oxo-2-tetrahydrofurancarboxylate.

17. The compound according to claim 1, which is 1-[(ethoxycarbonyl)oxy]ethyl 2-{(4S)-4-[(Z)-2-carbamoyl-2-fluorovinylthioacetamido]-3-oxo-2-isoxazolidinyl}-5-oxo-2-tetrahydrofurancarboxylate.

18. The compound according to claim 1, which is sodium 2-{(4S)-4-[(Z)-2-cyanovinylthioacetamido]-3-oxo-2-isoxazolidinyl}-5-oxo-2-tetrahydrofurancarboxylate.

19. A method of preparing a compound of the formula;

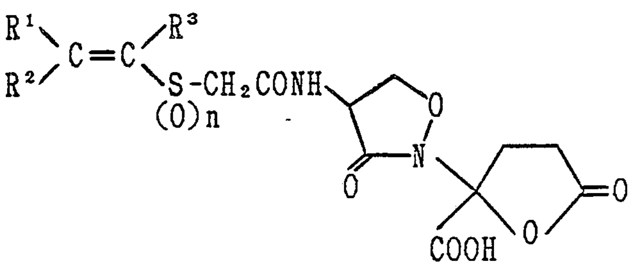

wherein $R^1$, $R^2$ and $R^3$ independently are hydrogen, halogen, cyano or carbamoyl, and n is an integer of 0, 1 or 2, a salt thereof or an ester thereof, which comprises reacting a compound of the formula;

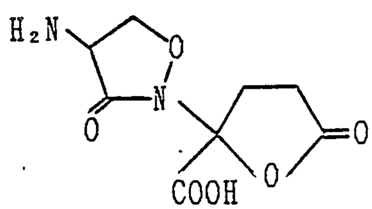

a salt thereof or an ester thereof with a compound of the formula;

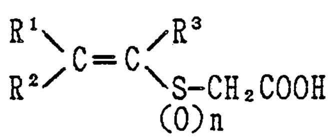

wherein $R^1$, $R^2$ and $R^3$ and n are of the meaning defined as above, or a reactive derivative at its carboxyl group, followed by, when desired, subjecting the reaction product to oxidation.

European Patent Office

# EUROPEAN SEARCH REPORT

Application number

EP 87 30 5067

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 88, no. 25, 19th June 1978, page 824, abstract no. 191406m, Columbus, Ohio, US; A. TSUJI et al.: "Reactive D-alanyl-D-alanine peptide derived from cycloserine", & HETEROCYCLES 1977, 8, 153-7 * Whole abstract * | 1 | C 07 K 5/06<br>A 61 K 37/02 |
| P,X | EP-A-0 191 989 (TAKEDA) * Claims * | 1 | |
| | | | **TECHNICAL FIELDS SEARCHED (Int. Cl.4)** |
| | | | C 07 K 5/00 |

The present search report has been drawn up for all claims

| Place of search | Date of completion of the search | Examiner |
|---|---|---|
| THE HAGUE | 27-08-1987 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82